# EUROPEAN PATENT APPLICATION

(11) **EP 0 608 497 A1**
(43) Date of publication of application: **03.08.1994**
(21) Application number: 93117615.0
(22) Date of filing: 29.10.1993
(51) Int. Cl.: A61L 15/18, A61L 15/42

(54) **Hypertonic saline dressing**

(30) Priority: 30.10.1992 US 968839
(71) Applicant: SPARTA SURGICAL CORPORATION, Hayward, California 94545 (US)
(72) Inventor: Reiner, Thomas F., San Ramon, California 94583 (US)
(74) Representative: Buchner, Otto, Dr.rer.nat.

(57) **Abstract**

A method of treating infected discharging wounds of the type wherein sterile therapeutically inert compress impregnated with an isotonic or hypertonic aqueous solution consisting essentially of sodium chloride is applied to the wound. The invention comprises impregnating a therapeutically inert compress with a predetermined quantity of a solution having a concentration of at least 18% by weight sodium chloride in sterile water. The concentration and the quantity in the compress are maintained until application thereof to the wound. The impregnated compress is then pressed against the wound and secured with a bandage.

## Description

### FIELD OF THE INVENTION

This invention relates to a method of treating wounds using a hypertonic dressing. More particular, the present invention relates to a dressing and a method of using the same, in which a high concentration of sodium chloride is impregnated in a sterile, therapeutically inert compress.

### BACKGROUND OF THE INVENTION

It has long been known that sodium chloride solution on wounds prevent the formation scabs or crust and keep the wound soft and pliable. Application of sodium chloride solution to wounds has become a standard practice, particularly for deep discharging or infected wounds. It is also a solution used as means of debridement of the wound.

As an improvement to avoid the difficulties involved in applying liquids or solutions to wounds, various wet dressings have been developed for topical therapy to relieve pain and promote healing. Typical wet dressings include pre-saturated sterile gauze dressings enclosed in convenient, aseptic peel open foil packages. Among the most effective are the wet saline dressings which use isotonic saline solutions, using a 0.9% normal saline solution dissolved in distilled water and sterilized, sold by Sparta Surgical Corporation.

Under some circumstances, use of saline wet dressings is not completely effective in solving all of the problems encountered in serious wounds, particular those which are opened and infected. Dressings can become uncomfortable, and require frequent changing of the dressing and the bed linen. With these isotonic solutions, if the wound is kept excessively wet, it encourages bacterial contamination, delays healing, and promotes maceration of the surrounding skin. If the dressing becomes dry, however, the risk is run that a scab would form over the infection, preventing healthy granulation tissue from being formed. Ideally, the saturated gauze should remain sufficiently most to allow formation of an epithelial bridge for most effective healing.

In an effort to overcome some of the difficulties of the prior art, a dry sodium chloride compress has been developed and is disclosed in U.S. Patent 4,608,044. This patent teaches the impregnation of a compress of the type used for treating infected discharging wound. The impregnated compress is first dried to evaporate substantially all of the water present to provide a dry compress. The dry compress is then pressed against the wound and is secured in place with a bandage. These dry sodium chloride compresses are applied to the wound with a spatula. The patent alleges that no maceration of the wound edges occurred and that pain was only experienced as an exception and for only a short time.

It has been found, however, that during the initial time when the dry sodium chloride compress has been applied, there is a risk of abrasion and irritation of the wound. Also, there is a time delay before the dry sodium chloride begins to draw the fluids surrounding the wound into the dressing. The dry sodium chloride dressing functions as a hypertonic dressing because the high salt content creates an osmotic effect, cleansing the wound and drawing or pulling exudate and necrotic tissue out of the wound into the dressing. It is believe that the dry dressing is limited in the way fluids are drawn into the dressing and also in the total quantity of fluid absorbed as equilibrium is reached. Accordingly, it is an object of this invention to provide a dressing with improved drawing power and improved capacity to draw fluid into the dressing and out of the wound.

Another object is to provide a dressing which reduces the discomfort experienced by the patient when it is applied and during it initial period of use.

Other objects will appear hereinafter.

### SUMMARY OF THE INVENTION

It has now been discovered that the above and other objects of the present invention maybe accomplished in the following manner. Specifically, an improvement in the method for treating infected discharging wounds and a compress for use in that method have been discovered.

It has been discovered that the method of treating infected discharging wounds of the type, wherein a sterile, therapeutically inert compress is impregnated with an aqueous solution of sodium chloride and applied to a wound, maybe improved. The improvement comprises impregnating a therapeutically inert compress with a predetermined quantity of a solution having concentration of at least 18% by weight sodium chloride in sterile water, maintaining the concentration and quantity of solution in the compress until application of the compress to the wound, and pressing the impregnated compress against wound and securing the compress with a bandage. Prior to use, the impregnated compress may be packaged in a barrier proof pouch and sterilized by one means or another, preferably gamma radiation.

The preferred concentration of sodium chloride in sterile water is about 20% and the preferred compress is gauze, either a single strip or a plurality of gauze layers. The method contemplates the application of a plurality of these compresses sequentially on a wound over a period of time until the wound discharge ceases, necrotic tissue is removed and health granulation tissue develops.

To accomplish the method of the present invention, a compress is provided. The compress, usually gauze and either one layer or several layers packaged together, is impregnated with a solution of at least 18% sodium chloride in sterile water. Preferably, the concentration is about 20% by weight sodium chloride in sterile water. The impregnated compress is then maintained in a ready condition, such as by packaging in barrier proof pouch and is then sterilized, preferably by gamma radiation, so that it is available for use.

### DETAILED DESCRIPTION OF THE PREFERRED EMBODIMENT

The present invention is an improvement over dry sodium chloride dressings. While both dry dressings and the improved sodium chloride wet dressing of this invention may be referred to as a hypertonic dressing, due to the osmotic effect created by the high salt content, the wet dressing of the present invention has been found to be surprisingly more effective. Osmotic dressings cleanse a wound by drawing or pulling exudate and necrotic tissue out of the wound and into the dressing. However, dry sodium chloride dressings are limited in wicking ability and absorptive capacity.

It is, of course, impossible to evaluate medical products on human beings without approval from the Federal Drug Administration (FDA). For that reason, clinical testing is not available at the time of the filing of this application. FDA approval has been received for this invention several days prior to the filing of this application. Approval of the FDA has been required even though isotonic (0.9%) sodium chloride saturated dressings have been approved for a long time and dry sodium chloride dressings have also received approval from the FDA. The requirement for the present invention that the FDA approve the method and the composition is due to the fact that the wet hypertonic sodium chloride dressing of the present invention is different in kind from the those previous approved by the FDA.

The following procedure has been approved for use of the present invention. The hypertonic sodium chloride wet dressing of this invention are to be used for chronic wounds in the inflammatory stages, particularly when wounds are deep, discharging or infected. The dressing of this invention are also indicated a means of debridement as part of the wet-to-dry dressing technique. The dressing is applied is directly onto the wound with edges loosely folded over to pack a deep wound or cover the wound surface only. Surrounding healthy skin should not covered if possible. A number of various sizes of hypertonic sodium chloride dressings have been prepared. Among these are:
7,6 x 7,6 cm (3" x 3"), 12 ply gauze pads;
10,2 x 10,2 cm (4" x 4"), 12 ply gauze pads;
1,3 x 101,6 cm (1/2" x 40") gauze strips; and
2,5 x 101,6 cm (1" x 40") gauze strip.

The gauze is U.S.P. Type VII gauze and sterilization is effected by gamma radiation. Samples were manufactured using 7,6 x 7,6 cm (3" x 3") 12 ply gauze pad impregnated with 3.8 grams of a 20% U.S.P. sodium chloride dissolved in U.S.P. distilled water. The pads were then sealed in a barrier proof pouch and sterilized for use. Other concentrations ranging from less than aobut 18% by weight to substantially more than 20% may be employed as well.

In the use of the dressing, it is intended that the pad be cover with a protected absorbent overdressing. Dressings should be changed one or twice daily, depending upon the amount of drainage. It is noted that treatment with hypertonic dressings of the present invention will reduce wound edema and debris, and also may result in an initial increase in wound size due to the removal necrotic tissue. Treatment is discontinued when wound discharge has ceased, necrotic tissue has disappeared, and health granulation tissue has developed.

In order to demonstrate the efficacy of the present invention, 3" X 3" hypertonic sodium chloride dressings were prepared as described above. These dressings were compared with dry sodium chloride impregnated dressings. Specifically, commercially available sodium chloride impregnated dressing formed from a rayon-polyester impregnated dressing have 15% (by weight) crystalline sodium chloride were obtained and used in the following experiments.

Dry sodium chloride dressings of the type described in U.S. Patent No, 4,608,044 have another major fault or drawback. Before these dressings can be used, the wound itself needs to have a quantity of isotonic saline solution poured on the wound as a base or foundation for the dressing. While this is not set forth in the patent, such instructions are provided with the commercially available products sold under this patent. Without the additional saline solution, the dressing fails to function as intended.

### EXPERIMENT I

The first set of experiments had as their objective to compare the hypertonic activity of the present invention with the dry sodium chloride dressing which is described in previously identified U.S. Patent No 4,608,044. In both cases, the sodium chloride present in the dressing functions to create a hypertonic environment or a osmotic pressure which is greater than the isotonic nature of blood and, therefore, the dressing draws the exudate in the wound toward the dressing. This helps clear the wound. Wicking ability or drawing power is therefore an important property of a sodium chloride dressing.

In a series of experiments, a one milliliter sample of crystal violet dye was placed on a piece of aluminum foil for each of the experiments. A wet sodium chloride dressing of the type described above, having a 20% sodium chloride solution and in accordance with the principles of this invention, was placed on sample of dye. A commercially available dry sodium chloride dressing was placed on another sample of dye. All weights have been accurately measured on an analytical balance. A dry 7,6 x 7,6 cm (3" x 3"), 12 ply gauze dressing was then placed on each of the sodium chloride dressings. These samples were made to sit for sixty seconds and the overlaid dressing was weighed accurately. The amount of dye which was transferred to the overlaid gauze pad was calculated for both experiments.

When the hypertonic dressing of the present invention was employed, 26% of the salt solution and dye were transferred to the overlaid pad in this sixty second test. When the commercially available dry pad the use, 2.01% of the dye was transferred. This result demonstrates that the present invention is surprisingly superior in hypertonic activity due to its substantially increased drawing power.

### EXPERIMENT II

It was also possible to evaluate the absorption of the present invention when compared to the dry dressing. In the following experiments 5,1 cm (2") diameter x 1,9 cm (3/4") high containers were filled with 25 milliliters of crystal violet dye solution. The dressing of this invention was placed on top the dye filled container and covered with 7,6 x 7,6 cm (3" x 3") pads.
Similarly, the commercially available dry hypertonic dressing and 7,6 x 7,6 cm (3" x 3") pads were placed upon a second container of dye. The impregnated dressing from each product plus the dry gauze pads were then plunged in the center with a stirring rod, so that the center of all the pads touched the pad of the container. The two containers with the dressings were allowed to absorb dye and reach equilibrium, namely that point where no more dye was absorbed, taking approximately 20 minutes to achieve equilibrium. At the end of the lapse time, the saturated dressing with the dye were removed and weighed. The amount of dye present was calculated.

The present invention was found to have absorbed 3.83 grams more dye than the commercially available dry sodium chloride pad. This represented approximately 17.5% more absorption for the present invention. Again, the present invention demonstrated a clear superiority over the prior commercially available product.

While particular embodiments of the present invention have been illustrated and described herein, it is not intended to limit the invention. There are changes and modifications being made therein within the scope of the following claims.

## Claims

1. In a method of treating infected discharging wounds of the type wherein a sterile, therapeutically inert compress impregnated with an isotonic or hypertonic aqueous solution consisting essentially of sodium chloride is applied to the wound, the improvement comprising:
impregnating a therapeutically inert compress with a predetermined quantity of a solution having a concentration of at least 18% by weight sodium chloride in sterile water;
maintaining said concentration and said quantity in said compress until application thereof to the wound; and
pressing the impregnated compress against the wound and securing the compress with a bandage.

2. The method of claim 1 wherein said impregnated compress is packaged in a barrier proof pouch and sterilized by gamma radiation for storage prior to use.

3. The method of claim 1 wherein said concentration is about 20% by weight sodium chloride in sterile water.

4. The method of claim 1 wherein said compress is formed from gauze.

5. The method of claim 4 wherein said compress comprises a plurality of gauze layers.

6. The method claim 1 wherein a plurality of said compresses are sequentially applied to the wound until the wound discharge ceases, necrotic tissue is removed and healthy granulation of tissue develops.

7. A compress for treating wounds of the type wherein a sterile, therapeutically inert compress impregnated in with isotonic or hypertonic aqueous solution consisting essentially of sodium chloride is applied to the wound comprising;
a therapeutically inert compress impregnated with a predetermined quantity of a solution having a concentration of at least 18% by weight sodium chloride in sterile water; and
a package for said compress suitable for maintaining said concentration and quantity and being sterilized by gamma radiation prior to use, said use including application of said impregnated compress against wound and securing the compress with a bandage.

8. The compress of claim 7 wherein said concentration of said sodium chloride in sterile water is about 20% by weight.

9. The compress of claim 7 wherein said compress is gauze.

10. The compress of claim 9 wherein said compress comprises a plurality of gauze layers.
